# EUROPEAN PATENT SPECIFICATION

(11) **EP 0 673 263 B1**
(45) Date of publication and mention of the grant of the patent: **26.02.2003**
(21) Application number: 94905386.2
(22) Date of filing: 10.12.1993
(51) Int. Cl.: A61M 1/00

(54) **QUICK DISCONNECT FOR LAPAROSCOPE PROBE TIP**
SCHNELLTRENNKUPPLUNG FÜR SONDENSPITZE EINES LAPARASKOPS
DISPOSITIF D'ACCOUPLEMENT A DECONNEXION RAPIDE POUR L'EXTREMITE DE SONDE D'UN INSTRUMENT DE LAPAROSCOPIE

(30) Priority: 11.12.1992 US 989109
(43) Date of publication of application: 27.09.1995
(73) Proprietor: C.R. BARD, INC., Murray Hill New Jersey 07974 (US)
(72) Inventor: DORSEY, James, H., III, Delray Beach, FL 33445 (US)
(74) Representative: Nette, Alexander
(86) International application number: US9312155
(87) International publication number: WO94013335

(56) References cited:
- EP-A- 0 457 220
- DE-U- 9 013 145
- US-A- 1 538 007
- US-A- 3 784 235
- US-A- 4 006 088
- US-A- 4 123 091
- US-A- 4 468 217
- US-A- 4 872 837
- US-A- 4 878 900
- US-A- 5 013 300

## Description

### 1. Field of the Invention

This invention relates to an interchangeable probe system. More specifically, this invention relates to a medical device for provision or delivery (infusion) of fluid and aspiration of fluid in relation to an operative field through an interchangeable probe operatively connected to such device. The interchangeable probe tip is coupled to a unique mount affixed to a handset of said device. This mount permits the rapid connection/disconnection of an interchangeable probe and freedom of orientation of the probe, relative to the handset, while preserving the sealing engagement thereof to the handset.

### 2. Description of the Prior Art

The provision of medical service involving surgical procedures has and continues to tax the limits of the health services industry, which includes the limited availability of skilled professionals to deliver such services and the individual's and insurer's ability/willingness to pay for extended periods of recovery int he supervised environment of a hospital. A prolonged recuperative period also causes potential economic hardship to the patient in the nature of lost earnings and exhaustion of medical benefits. Accordingly, there has and continues to be an emphasis on the delivery of health related services on an outpatient basis or by the adoption of techniques which reduce the burden on the health service industry and the patient. One such evolution has occurred in the surgical field through the implementation of "least invasive surgical" techniques (also hereinafter designed "LIS techniques" or "laparoscopy") in place of the traditional "open" or "large incision" oriented surgical procedures.

The rapid adoption and popularity of laparoscopy (surgery through micro incisions and with the aid of fiberoptics) is without precedent in modern surgical history. Surgeons throughout the world have eagerly embraced this operation, primarily because of the significant benefits in overall patient care that laparoscopic surgery offers.

Until recently, laparoscopy was performed strictly by gynecologists and not general surgeons or other specialists. During the late 1980's, this started to change when a general surgeon pioneering a new field began to perform cholecystectomy (removal of the gallbladder) laparoscopically. This technique quickly became the norm. With laparoscopic surgery gaining widespread acceptance, more and more procedures that were previously performed with conventional large, painful and temporarily disabling incisions, started being performed utilizing the laparoscopic modality. This field is still at a rapid growth rate. The need for new instruments to perform these procedures is great and the lack of such instruments is a limiting factor in the changeover from open to endoscopic surgical procedures.

Due to the lack of specifically designed instruments for general surgical laparoscopic procedures, new general surgical laparoscopists were forced to use existing gynecologic instrumentation. The predecessor to the modern medical device utilized in both laparoscopic and general surgery for infusion and aspiration of fluids were, thus, based upon devices originally designed for gynecological procedures. One instrument that was adapted for general surgical use was the "suction/irrigator." Various other electrosurgical instrumentation was also utilized. Electrosurgical instruments consisted of an insulated solid rod or hollow cannula with an exposed electrosurgical tip of several different configurations. These could be utilized to both bluntly and electrosurgically (cutting with electricity) to dissect the gallbladder from the liver bed. Electrosurgical energy can also be utilized to stop bleeding. Typically, cannula type electrosurgical instruments were equipped with one trumpet valve which was used for the evacuation of smoke (a byproduct of electrosurgery) that is created during electrosurgical dissection. The inherent protrusion of the extended tip from the electrosurgical instrument and the small internal diameter limited suction capabilities. Also, the Luer Lock connector utilized on these instruments would clog due to the Luer's small internal diameter and restrictive nature and thus waste surgical time. To switch back to a non-obstructive suction irrigation cannula was time consuming.

To satisfy the needs of the industry a symmetrical hand actuated trumpet valve which incorporated an interchangeable probe tip concept onto a laparoscopic suction/irrigator was developed. Prior to this, all suction/irrigators were one piece (the probe tip being permanently affixed to the handset or valve control body).

The instant invention as disclosed is a basic assembly which further facilitates endoscopic surgery. The invention of interchangeable probe systems is nowhere disclosed in the prior art. Because of the previous obscurity of laparoscopic surgical procedures (limited to gynecological procedures), the prior art has had little or no use for this type of instrument assembly beyond its original field of application, nor any need or incentive to modify it to accommodate a field of use that was as yet to emerge.

In order to gain perspective into the evolution of the field of laparoscopy and the techniques attendant thereto, it is initially necessary to first gain an appreciation of the instrumentation originally associated with this field. The valve body traditionally associated with laparoscopic instruments contained either two trumpet style valves or two stop cocks which were used to control suction and fluid flow. A cannula (hollow tube) was attached to the body which housed these valves. During laparoscopy, the cannula would be inserted into the abdominal cavity to enable suction and irrigation of fluids. Dependent upon physician preference, these cannula were configured either with or without holes on the tip and also were available in several lengths. A cannula with holes would enable more efficient suction of fluids and debris with less clogging. A cannula without holes allowed a physician to utilize water pressure as a dissecting force (i.e. hydro-dissection) and also allowed for the suction retraction of tissue. If, during a procedure, a different tip design was necessary, the total unibodied design handpiece with cannula was changed. Luer Lock connectors (utilized in the medical field for fluid connection) allowed for this, but with this design a new suction irrigator had to be used in order to utilize a different cannula, as Luer Lock connectors are an integral part of the valve. Thus, it necessitated complete trumpet valve disconnection prior to changing probe tip/cannula design (either length or tip configuration) and turning off the irrigation source during this changing period.

The symmetrical trumpet valve concept consists of a versatile instrument for control of suction and irrigation that allows the utilization of all types of cannula and probe tips, both insulated and non-insulated in conjunction with a valve body/handset of symmetrical design. Probes with varying diameters, lengths and also electrosurgical cannula are, thus, able to be adapted to a hand-held housing which could control suction and irrigation functions. These attachments are easily and expeditiously changed without interruption of either the suction or irrigation source. In connection with this purpose a threaded connection, as for instance shown in EP-A-0 457 220, was developed. This concept allows utilization of many different attachments to one universal body.

However, threading and unthreading attachments is not as expeditiously accomplished as is possible. Threads can be misaligned and time can be wasted during the surgical procedure as attachments are changed. Because of the foregoing limitation, new methods for quickly attaching and detaching interchangeable probe tips and attachments to the body of the trumpet valve are needed. Furthermore, in order to fulfill the demanding requirement of the laparoscopic surgeon, a quick disconnect assembly which allows for 360 degree rotation of the attachment for varied orientation of electrosurgical tips during use in the surgical environment is further required. Such preference of the clinician for freedom of orientation need occur without rotation of the body of the trumpet valve. Additional requirements of the surgeon demand that this quick disconnect fitting will accommodate five or ten millimeter outside diameter cannula probe tips or electrosurgical attachments and permit coupling to either end of the symmetrical valve.

As is evident from the foregoing discussion, the increasing adaptation of laparoscopic surgical procedures to operations traditionally requiring a large incision has introduced a whole new set of demands and specifications for the instrument designer and the surgeon for whom it is designed. Up to now, those demands have been only partially fulfilled with the introduction of the trumpet valve; and there continues to exist need for improvement thereto to further enhance the versatility and ease of use of the trumpet valve by providing greater ease and speed of interchange of probe tips to the trumpet valve body.

### SUMMARY OF THE INVENTION

It is the object of this invention to remedy the above and related deficiencies in the prior art.

It is the principal object of this invention to provide an adaptor for use in conjunction with a medical device which permits the rapid attachment and disengagement of a probe to the medical device.

It is another object of this invention to provide an adaptor for rapid attachment and disengagement of a probe to a medical device, which device permits for infusion and aspiration of fluid through such probe to an operative field.

It is yet another object of this invention to provide an adaptor for rapid attachment and disengagement of an electrosurgical probe to a medical device, which device permits the delivery of electrosurgical current through said probe to an operative field.

It is still yet another object of this invention to provide an adaptor for both rapid attachment and disengagement of a probe to a medical device and the articulation of such probe, relative to the medical device, while maintaining sealing engagement thereof to the adaptor and the medical device.

Another object of this invention is to provide 360 degree rotation of electrosurgical attachments to facilitate endoscopic dissection.

Additional objects of this invention include providing a unique adaptor of this invention formed as a component of a medical device, and the use thereof in conjunction with interchangeable probe tips.

The above and related objects are achieved by providing, in combination, a valve assembly designed for use in conjunction with a probe tip and an adaptor, in operative association therewith, whereupon the adaptor provides (i) for operative and rapid engagement and disengagement thereof with one or more of the probe mounts to the medical device.

In one of the preferred embodiments of this invention, the adaptor comprises three basic or principal components; (a) a male connector for mounting of an interchangeable probe tip; (b) a female connector for mounting of the adaptor to the body of the medical device; and c) an articulating seal associated with the male connector for coupling to the probe tip. It is understood that in each of the components of the adaptor, a male connector can be used in lieu of a female connector and a female connector in lieu of a male connector consistent with the design parameters of the valve body and probe tip to which they intended to mate.

Other objects and advantages of this invention will become apparent from the following description taken in conjunction with the accompanying drawings wherein set forth, by way of illustration and example, certain embodiments of this invention. The drawings constitute a part of this specification and include exemplary embodiments of the present invention and illustrate various objects and features thereof.

### BRIEF DESCRIPTION OF THE DRAWINGS

The invention may be better understood by reference to the drawings in which:
Figure 1 is a perspective view of the medical device of the invention equipped with an interchangeable electrosurgical probe tip;
Figure 2 is an exploded view of the medical device of Figure 1, illustrating the individual components thereof;
Figure 3 is a partial section through the medical device of Figure 1 at the junction of coupling of a probe tip and the handset (trumpet valve) thereof:
Figure 4 is an exploded view of a medical device (not an embodiment of the present invention) illustrating the individual components;
Figure 5 is a partial section through the medical device of Figure 4 at the junction of coupling of a probe tip and the handset (trumpet valve) thereof;
Figure 6 is an exploded view of a second embodiment of the present invention illustrating the individual components;
Figure 7 is a partial section through the medical device of Figure 6 at the junction of coupling of a probe tip and the handset (trumpet valve) thereof;
Figure 8 is a perspective view of the medical device of Figure 1 illustrating the freedom of rotation of the probe tip relative to the handset while maintaining sealing engagement therebetween;
Figure 9 is a perspective view showing a prior art pistol grip valve incorporated with the interchangeable probe capabilities of the present invention;
Figure 9a is a perspective view showing the prior art pistol grip of Figure 9 and showing a partial section view of the interchangeable probe portion of Figure 9; and
Figure 10 is a perspective view of interchangeable suction/irrigation probes of differing shapes and orientation adapted for rapid configuration with the handset.

### DESCRIPTION OF THE PREFERRED EMBODIMENTS

The following description is made in reference to one or more of the accompanying drawings. Where a component appears in more than one drawing, it is assigned a common reference numeral for continuity of expression and ease of understanding.

The basic configuration of the trumpet valve concept is described in US-A-5 188 591.

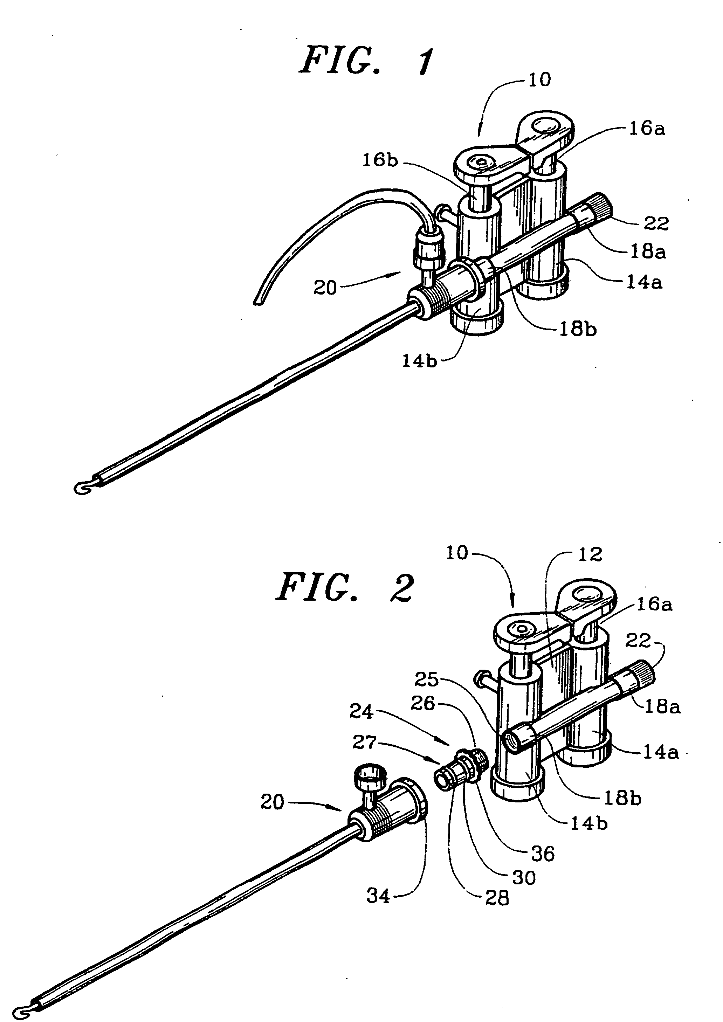

Figures 1 through 3 illustrate a first embodiment of the improved medical device which includes a trumpet valve generally shown at 10. The valve consists of a valve housing or body 12 which defines a pair of valve chambers 14a and 14b adapted for reciprocating movement of a pair of pistons 16a and 16b. The original trumpet valve concept, as described and claimed in the above-referenced commonly assigned patent, is basically symmetrical in overall appearance. The basic operation of the improved trumpet valve of the present invention is essentially the same as set forth in the above-referenced patent. More specifically, trumpet valve 10 is initially connected to a source of pressurized fluid and to a source of negative pressure via a pair of fittings (not shown) which extend from the valve body. In addition, the clinician/surgeon mounts an interchangeable probe tip, generally shown at 20, onto either one or two positions 18a or 18b on valve body 12 designed for this purpose. The position not having probe tip 20 mounted thereto will have a cap 22 mounted thereto. Once valve 10 is assembled and connected to the pressurized source of fluid and negative pressure through the fittings it is ready for use.

Typically, valve 10 can be used in a hydrodissection procedure or in conjunction with other laparoscopic instruments to perform various types of biopsy or infusion of irrigation fluid to clear the operative field and thereby allow for free and unobstructive access and view.

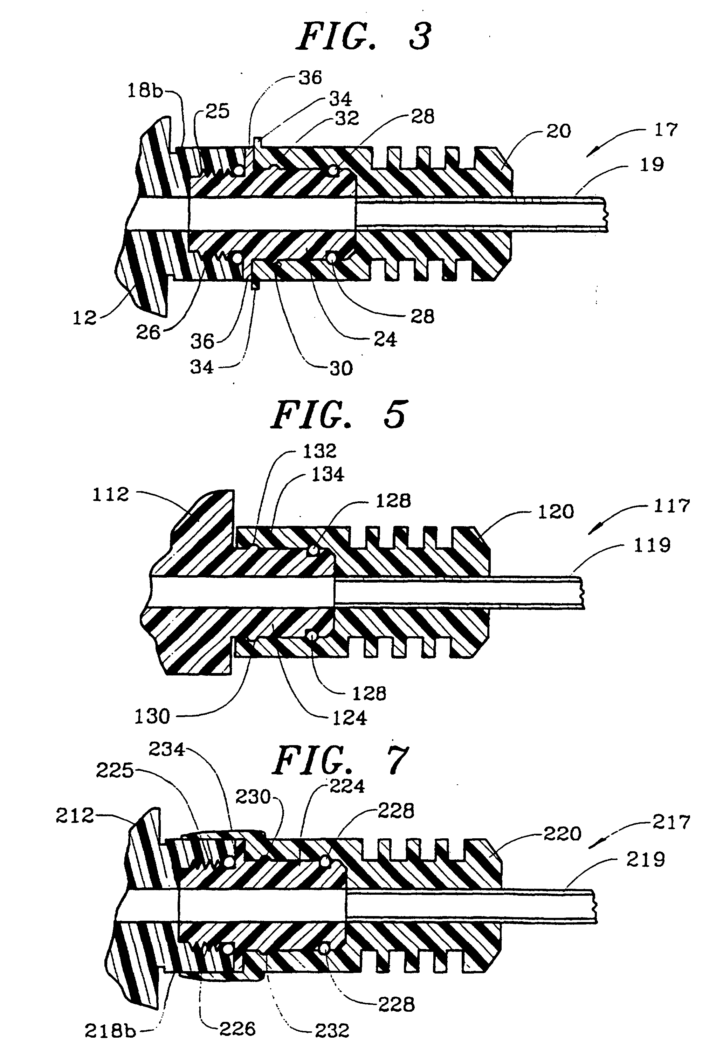

Figure 2 is an exploded view of the medical device of Figure 1, illustrating the individual components thereof. More specifically, the three components of the medical device and their relationship to one another reveals the basic valve body 12 having an adaptor receiving means. As seen in Figure 3, receiving means 25 can be a threaded orifice or aperture defined at either or both ends of valve body 12 for receiving the first end of adaptor 24. The threaded orifice allows 25 for threading engagement with a complementary thread 26 of the first end of an adaptor 24. In the specific embodiment of this mating, adaptor 24 is removeably but securely engaged with valve body 12. The second end of adaptor 24 is provided for operative and rapid engagement and disengagement thereof with a probe 17. The probe 17 includes a probe shaft 19 which is housed at a first end by a probe shaft connector 20. Connector means 20 is a specially designed housing encompassing the first end of shaft 19 and is operatively associated with the second end of adaptor 24. When the first end of adaptor 24 is removeably secured to receiving means 25, adaptor 24 functions as a male protruding member for removable engagement with a female adaptor receiving cavity of connector means 20. As the probe mount is easily and quickly removable from adaptor, numerous probe mounts, having various probe tip configurations and diameters, can readily be interchange, without replacing valve body 12 and adaptor 24, for various situations which can arise during surgery.

Figure 3 is an enlarged cross sectional view in partial of a section of trumpet valve 10 showing adaptor 24 having threads 26 and removeably connected to a probe shaft connector 20 having probe tip 21 and to adaptor receiving means 25 on valve body 12. As adaptor 24 has threads 26 at its first end it can be used with new or existing prior art valve bodies. As seen in Figure 3, adaptor 24 is threaded on a first end (or distal portion thereof) which is removeably connected to the receiving means portion 25 of valve body 12 and modified in a somewhat different fashion of a portion thereof which is designed for rapid coupling and de-coupling to an interchangeable probe 17 having a probe tip 21. More specifically, adaptor 24 is further provided with complementary snap-lock seal, generally shown at 27, on the proximal end thereof including means for sealing engagement with probe shaft connector 20 and means for snap lock engagement and disengagement at the proximal end of probe 17 with adaptor 24.

As seen in Figures 2 and 3 the seal 27 is in the form of a ring 28 mounted near the second end of adaptor 24. Preferably, ring 28 is constructed from silicone. The snap lock engagement/disengagement of probe 20 to adaptor 24 is provided by complementary detente, molded into collar 30 and groove 32 on the mating part of adaptor 24 and probe 20, respectively, to provide for physical retention of probe 20 to adaptor 24 during use and the quick release thereof when the clinician desires to interchange one probe tip for another. Furthermore, collar 34 of probe shaft connector 20 is shown flush against collar 36 of adaptor 24 to provide an additional seal between adaptor 24 and probe shaft connector 20. Collar 36 of adaptor 24 is also shown flush against the end of adaptor receiving means 25 to provide an additional seal between adaptor 24 and adaptor receiving means 25. Seal means 27, which prevents leakage of probe shaft connector 20 once mounted to adaptor 24, can be located on or within the probe itself in lieu of on adaptor 24 or both adaptor 24 and probe shaft connector 20 can contain separate yet complementary sealing means. Seal means 27 can be an o-ring 28 disposed on the outer surface portion of the second end of adaptor 24. O-ring 28 can be associated with a second groove defined by the inner surface portion of connector means 20 or can be associated with the inner surface portion of connector means 20 without the second groove. Alternatively, seal means 27 can be an o-ring 28 disposed on the inner surface portion of connector means 20. In this alternative embodiment for seal means 27, o-ring 28 is associated with a groove defined by the outer surface portion of the second end of adaptor 24 or is associated with the outer surface portion without the groove. Though probe 17 is shown mounted to fitting 18b and cap 22 is shown fitted to fitting 18a, the invention is not limited to this configuration and probe 17 can be used mounted to fitting 18a while cap 22 is mounted to fitting 18a. Thus, the present invention can be easily used by either a right or left hand user.

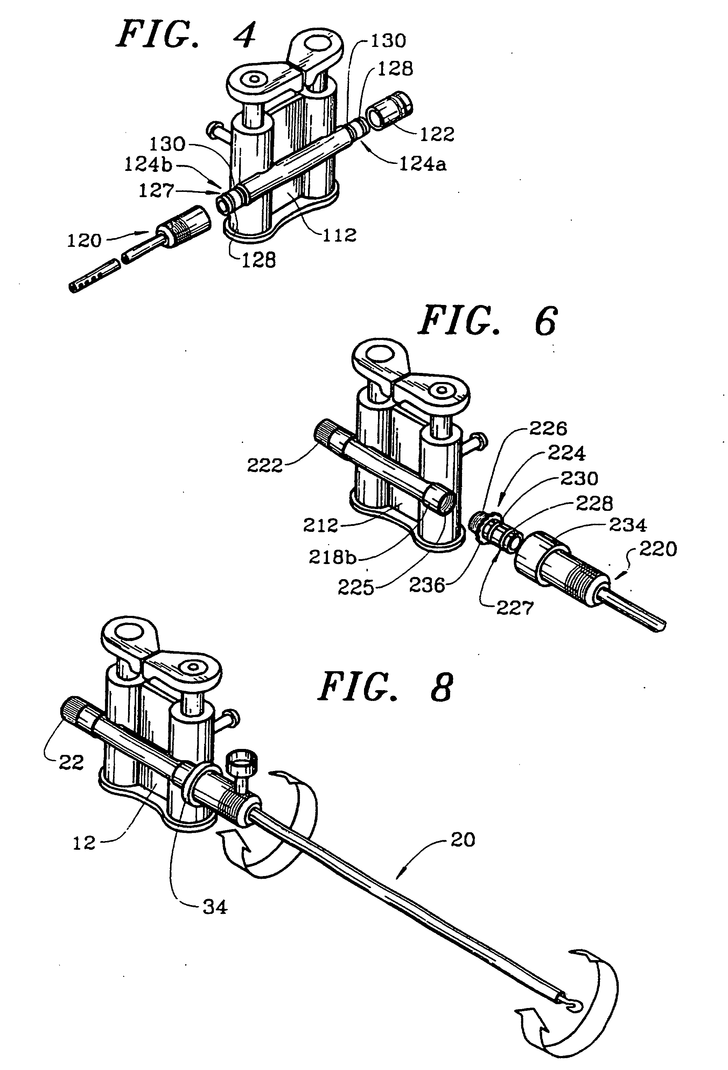

In Figures 4 and 5, valve body 112 operates and is constructed similar to valve body 12 of the first embodiment with one exception. As seen in the drawings, the probe 117 includes a probe shaft 119 which is housed at a first end by a probe shaft connector 120. Connector means 120 is a specially designed housing encompassing the first end of shaft 119 and is operatively associated with the second end of valve interface means or adaptor 124. Interface means 124 is a male protruding member constructed integral with valve body 112 for removeable engagement with a female interface receiving cavity of connector means 120. Interface means 124 is constructed integral with valve body 112. As with the first embodiment, the second end of interface means 124 is provided for operative and rapid engagement and disengagement thereof with a probe shaft connector 120. Probe shaft connector 120 is connected to interface means 124 essentially identical to probe shaft connector 20 and adaptor 24 of the first embodiment. Thus, interface means 124 is provided with a complementary snap-lock seal, generally shown at 127, which includes means for sealing engagement with probe shaft connector 120 and means for snap lock engagement and disengagement between probe shaft connector 120 and interface means 124.

Seal means 127 can be an o-ring 128 disposed on the outer surface portion of the interface means 124. O-ring 128 can be associated with a second groove defined by the inner surface portion of connector means 120 or can be associated with the inner surface portion of connector means 120 without the second groove. Alternatively, seal means 127 can be an o-ring 128 disposed on the inner surface portion of connector means 120. In this alternative embodiment for seal means 127, o-ring 128 is associated with a groove defined by the outer surface portion of the interface means 124 or is associated with the outer surface portion without the groove.

Seal 127 is in the form of a ring 128 mounted near the end of interface means 124. The snap lock engagement/disengagement of probe shaft connector 120 to interface means 124 is provided by complementary detente, molded into collar 130 and groove 132 on the mating part of interface means 124 and probe shaft connector 120, respectively, to provide for physical retention of probe shaft connector 120 to interface means 124 during use and the quick release thereof when the clinician desires to interchange one probe tip for another. Furthermore, probe shaft connector 120 is shown substantially encompassing the entire length of interface means 124 to provide an additional seal between interface means 124 and probe shaft connector 120. Seal means 127, which prevents leakage of probe shaft connector 120 once mounted to interface means 124, can be located on or within the probe itself in lieu of on interface means 124 or both interface means 124 and probe shaft connector 120 can contain separate yet complementary sealing means. A cap 122 is provided at the end of valve body 112 not having probe 120 mounted thereto. By having the capability of mounting probe shaft connector shaft connector 120 at either end of valve body 112, the device shown in Figures 4 and 5 can be used either for right or left handed use.

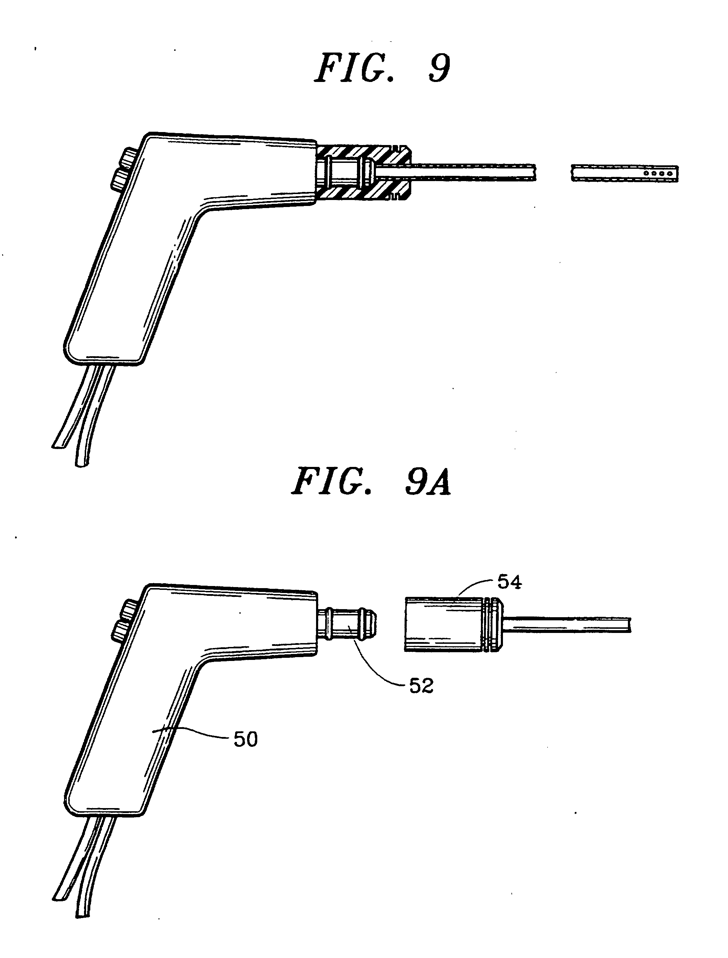

Figures 6 and 7 illustrate a second embodiment of the present invention. In this embodiment, valve body 112 is operates and is constructed similar to valve body 12 of the first embodiment. Valve body 212 includes an adaptor receiving means 225. Receiving means 225 can be a threaded orifice or aperture defined at either or both ends of valve body 212 for receiving the first end of adaptor 224. In this second embodiment, adaptor 224 is similar to adaptor 24 of the first embodiment and is removeably but securely engaged with valve body 212. As seen in the drawings, the probe 217 includes a probe shaft 219 which is housed at a first end by a probe shaft connector 220. Connector means 220 is a specially designed housing encompassing the first end of shaft 219 and is operatively associated with the second end of adaptor 224. The first end of adaptor 224 is threaded to allow for removeable securement to receiving means 225 of valve body 212. When the first end of adaptor 224 is removeably secured to receiving means 225, adaptor 224 functions as a male protruding member for removeable engagement with a female adaptor receiving cavity of connector means 220. The second end of adaptor 224 is provided for operative and rapid engagement and disengagement thereof with probe shaft connector 220.

Adaptor 224 is designed for rapid coupling and de-coupling to an interchangeable probe 217. More specifically, adaptor 224 is further provided with complementary snap-lock seal, generally shown at 227, on the proximal end thereof including means for sealing engagement with probe shaft connector 220 and means for snap lock engagement and disengagement at the proximal end of probe shaft connector 220 with adaptor 224.

Seal means 227 can be an o-ring 228 disposed on the outer surface portion of the second end of adaptor 224. O-ring 228 can be associated with a second groove defined by the inner surface portion of connector means 220 or can be associated with the inner surface portion of connector means 220 without the second groove. Alternatively, seal means 227 can be an o-ring 228 disposed on the inner surface portion of connector means 220. In this alternative embodiment for seal means 227, o-ring 228 is associated with a groove defined by the outer surface portion of the second end of adaptor 224 or is associated with the outer surface portion without the groove.

Seal 227 is in the form of a ring 228 mounted near the second end of adaptor 224. The snap lock engagement/disengagement of probe shaft connector 220 to adaptor 224 is provided by complementary detente, molded into collar 230 and groove 232 on the mating part of adaptor 224 and probe shaft connector 220, respectively, to provide for physical retention of the interchangeable probe 217 to adaptor 224 during use and the quick release thereof when the clinician desires to interchange one probe tip for another. Furthermore, probe shaft connector 220 is shown substantially encompassing the entire length of adaptor 224 and fitting 218b to provide an additional seal between adaptor 224 and probe shaft connector 220. Collar 236 of adaptor 224 is also shown flush against the end of fitting 218b to provide an additional seal between adaptor 224 and adaptor receiving means 225. Seal means 227, which prevents leakage of the interchangeable probe 217 once mounted to adaptor 224, can be located on or within the probe shaft connector itself in lieu of on adaptor 224 or both adaptor 224 and probe shaft connector 220 can contain separate yet complementary sealing means. Though the interchangeable probe 217 is shown mounted to fitting 218b and cap 222 is shown fitted to fitting 218a, the invention is not limited to this configuration and the interchangeable probe 217 can be used mounted to fitting 218a while cap 222 is mounted to fitting 218b. Thus, the present invention can be easily used by either a right or left hand user.

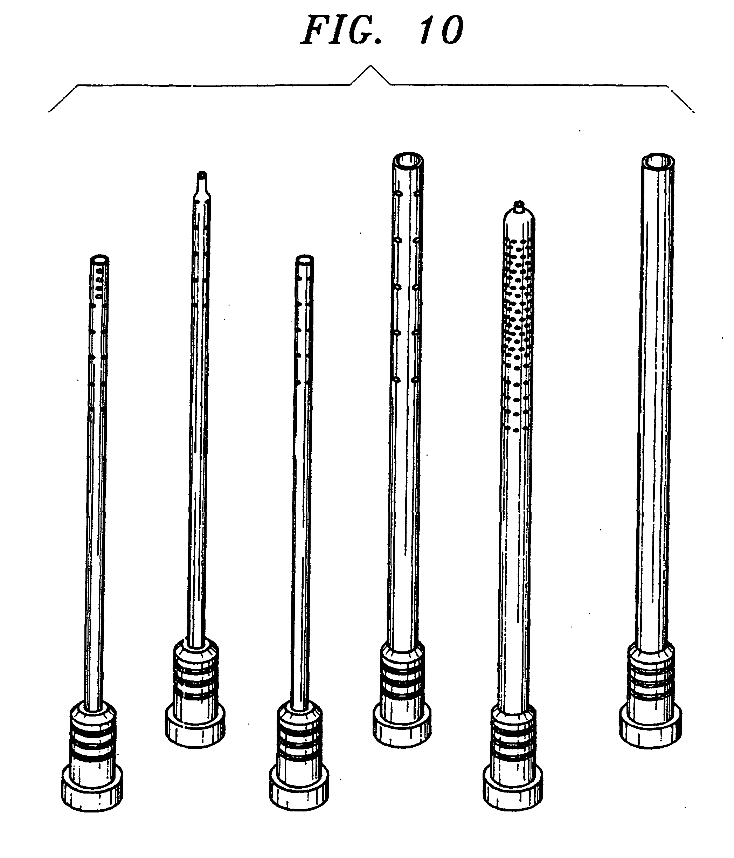

As the probe mount is easily and quickly removable from adaptor, numerous probe mounts, having various probe tip configurations and diameters, can readily be interchange, without replacing valve body and adaptor, for various situations which can arise during surgery. A representative sample of such interchangeable probe mounts are illustrated in Figure 10. However, numerous other probe mounts not shown can also be used with the present invention.

Figure 8 illustrates the articulation of electrosurgical probe in relation to the handset. Such articulation is possible due to the sealing engagement/coupling of the electrosurgical probe 20 to the unique adaptor 24 of this invention. Thus, probe 20 is allowed to rotate freely 360 degrees with respect to valve body 12 and adaptor 24 while retaining its sealed relationship with adaptor 24 at all times.

Figures 9 and 9a illustrates the concepts of quick disconnect probe tip of the present invention in conjunction with a prior art pistol type grip valve 50. Adaptor 52 is removeably connected to valve 50. Probe 54 sealably connects to adaptor 52 in a similar manner as described above for the various embodiments shown in the drawings.

The foregoing describes a number of representative and preferred embodiments of this invention. It is not the purpose and intent of the foregoing to, however, delineate the scope of the invention which is set forth in the claims appended hereto. Further, it should be recognized that numerous other probe tip configurations and diameters, not shown herein, may also be used with the concepts of the present invention.

It is to be understood that while it has been illustrated and described certain forms of the invention, it is not to be limited to the specific forms or arrangement or parts herein described and shown. It will be apparent to those skilled in the art that various changes may be made without departing from the scope of the invention and the invention is not to be considered limited to what is shown in the drawings and described in the specification.

## Claims

1. An interchangeable probe system for rapid removal or attachment of a surgical probe (17, 217) to a valve assembly (10), said valve assembly defining an internal conduit for fluid flow having a first end and a second end, said interchangeable probe system comprising:
said surgical probe (17, 217) including a probe shaft (19, 219), said surgical probe further including a connector means (20, 220), said connector means secured to said probe shaft;
said valve assembly including an interface means disposed at one end of said conduit;
said interface means and said connector means operatively associated with one another for providing rapid engagement and disengagement of a surgical probe to said valve assembly; and
the interface means comprises an adaptor means (24, 224) having a first end and a second end, said adaptor means operatively threadably associated with a receiving means (25, 225) of said valve assembly (10) at said first end and means for providing a snap lock engagement and disengagement between said interface means and said connector means at said second end.

2. The interchangeable probe system of claim 1, wherein said connector means (20, 220) houses a portion of said probe shaft (19, 219).

3. The interchangeable probe system of claim 1 or 2, wherein said means for providing a snap lock engagement is a groove (32, 232) defined by an inner surface portion of said connector means (20, 220) and a corresponding collar (30, 230) disposed on an outer surface portion of said interface means , said groove and said collar operatively associated with each other to provide a frictional engagement between said connector means and said interface means.

4. The interchangeable probe system of claim 1, 2 or 3 further including sealing means (27, 227) for preventing the passage of liquid between said interface means and said connector means (20, 220).

5. The interchangeable probe system of claim 4, wherein said sealing means is an o-ring (28, 228) assembly including a notch defined by an outer surface portion of said interface means and a second groove defined by an inner surface portion of said connector means (20, 220), said notch and second groove housing an o-ring (28, 228), when said connector means is removeably secured to said interface means said o-ring assembly providing a sealed and aligned relationship between said connector means and said interface means.

6. The interchangeable probe system of claim 1 further including second interface means disposed at the opposite end of the internal conduit to allow engagement of said connector means (20, 220) at either end of the conduit.

7. The interchangeable probe system of claim 1, wherein an inner surface portion of said receiving means (25, 225) having a female threaded member.

8. The interchangeable probe system of claim 7, wherein an outer surface portion of the first end of said adaptor means (24, 224) having a male threaded member (26, 226) for mating with the threaded inner surface portion of said receiving means (25, 225) when said adaptor means is removeably secured to said receiving means.

## Patentansprüche

1. Ein austauschbares Probensystem zur schnellen Entfernung oder Anbringen einer chirurgischen Probe (17, 217) an eine Ventileinrichtung (10), wobei die Ventileinrichtung eine innere Durchführung für einen Fluidfluss mit einem ersten Ende und einem zweite Ende bestimmt, und wobei das austauschbare Probensystem umfasst: die chirurgische Probe (17, 217), die einen Probenschaft (19, 219) umfasst, die chirurgische Probe ferner ein Verbindungsmittel (20, 220) umfasst, und das Verbindungsmittel an dem Probenschaft angebracht ist;
die Ventileinrichtung ein Grenzflächenmittel umfasst, das an einem Ende der Durchführung angeordnet ist;
das Grenzflächenmittel und das Verbindungsmittel betriebsmäßig miteinander verbunden sind zum schnellen Ineingriffbringen und Lösen einer chirurgischen Probe und der Ventileinrichtung; und
das Grenzflächenmittel ein Adaptormittel (24, 224) mit einem ersten Ende und einem zweiten Ende aufweist, das Adaptormittel betriebsmäßig und gewindemäßig mit einem Aufnahmemittel (25, 225) der Ventileinrichtung (10) an dem ersten Ende verbunden ist, und ein Mittel zum Vorsehen eines Arretiereingriffes und Lösen desselben zwischen dem Grenzflächenmittel und dem Verbindungsmittel an dem zweiten Ende aufweist.

2. Das austauschbare Probensystem nach Anspruch 1, wobei das Verbindungsmittel (20, 220) einen Abschnitt des Probenschaftes (19, 219) unterbringt.

3. Das austauschbare Probensystem nach Anspruch 1 oder 2, wobei das Mittel zum Vorsehen eines Arretiereingriffes eine Nut (32, 232) ist, die von einem inneren Oberflächenabschnitt des Verbindungsmittels (20, 220) und einem entsprechenden Bund (20, 230), der an einem äußeren Oberflächenabschnitt des Grenzflächenmittels angeordnet ist, bestimmt ist, die Nut und der Bund betriebsmäßig miteinander verbunden sind, um einen reibungsmäßigen Eingriff zwischen dem Verbindungsmittel und dem Grenzflächenmittel vorzusehen.

4. Das austauschbare Probensystem nach Anspruch 1, 2 oder 3, desweiteren umfassend ein Abdichtmittel (27, 227) zum Verhindern des Durchflusses von Flüssigkeit zwischen dem Grenzflächenmittel und dem Verbindungsmittel (20, 220).

5. Das austauschbare Probensystem nach Anspruch 4, wobei das Abdichtmittel eine O-Ring-(28, 228)-Einrichtung ist, die eine Kerbe, die von einem äußeren Oberflächenabschnitt des Grenzflächenmittels bestimmt ist, und eine zweite Nut umfasst, die von einem inneren Oberflächenabschnitt des Verbindungsmittels (20, 220) bestimmt ist, wobei die Kerbe und die zweite Nut einen O-Ring (28, 228) unterbringt, wenn das Verbindungsmittel auf entfernbare Weise an dem Grenzflächenmittel angebracht ist, und wobei die O-Ring-Einrichtung eine abgedichtete und ausgerichtete Beziehung zwischen dem Verbindungsmittel und dem Grenzflächenmittel vorsieht.

6. Das austauschbare Probensystem nach Anspruch 1, desweiteren umfassend ein zweites Grenzflächenmittel, das an dem gegenüberliegenden Ende der inneren Durchführung angeordnet ist, um das Ineingriffbringen des Verbindungsmittels (20, 220) an einem der beiden Enden der Durchführung zu ermöglichen.

7. Das austauschbare Probensystem nach Anspruch 1, wobei ein innerer Oberflächenabschnitt des Aufnahmemittels (25, 225) ein weibliches Gewindeelement aufweist.

8. Das austauschbare Probensystem nach Anspruch 7, wobei ein äußerer Oberflächenabschnitt des ersten Endes des Adaptormittels (24, 224) ein männliches Gewindeelement (26, 226) aufweist, das mit dem inneren Oberflächenabschnitt des Aufnahmemittels (25, 225), der ein Gewinde aufweist, zusammenwirkt, wenn das Adaptormittel auf entfernbare Weise an dem Aufnahmemittel angebracht ist.

## Revendications

1. Système de sonde interchangeable pour retirer ou fixer rapidement une sonde chirurgicale (17, 217) par rapport à un ensemble formant valve (10), ledit ensemble formant valve définissant un conduit interne pour un écoulement de liquide ayant une première extrémité et une seconde extrémité, ledit système de sonde interchangeable comprenant :
ladite sonde chirurgicale (17, 217) qui comprend un axe de sonde (19, 219), ladite sonde chirurgicale comprenant en outre des moyens de raccordement (20, 220), lesdits moyens de raccordement étant fixés audit axe de sonde ;
ledit ensemble formant valve comprenant des moyens d'interfaçage disposés à une extrémité dudit conduit ;
lesdits moyens d'interfaçage et lesdits moyens de raccordement étant associés de manière fonctionnelle les uns aux autres pour fournir une mise en prise et une désolidarisation rapides d'une sonde chirurgicale avec ledit ensemble formant valve ; et
les moyens d'interfaçage comprenant des moyens formant adaptateur (24, 224) ayant une première extrémité et une seconde extrémité, lesdits moyens formant adaptateur étant associés de manière fonctionnelle et par filetage à des moyens de réception (25, 225) desdits moyens formant valve (10) au niveau de ladite première extrémité et des moyens pour fournir une mise en prise et une désolidarisation par encliquetage entre lesdits moyens d'interfaçage et lesdits moyens de raccordement au niveau de ladite seconde extrémité.

2. Système de sonde interchangeable selon la revendication 1, dans lequel lesdits moyens de raccordement (20, 220) renferment une partie dudit axe de sonde (19, 219).

3. Système de sonde interchangeable selon la revendication 1 ou 2, dans lequel lesdits moyens fournissant une mise en prise par encliquetage est une gorge (32, 232) définie par une partie de surface intérieure desdits moyens de raccordement (20, 220) et un collier correspondant (30, 230) disposé sur une partie de surface extérieure desdits moyens d'interfaçage, ladite gorge et ledit collier étant associés de manière fonctionnelle l'un à l'autre pour fournir une mise en prise par frottement entre lesdits moyens de raccordement et lesdits moyens d'interfaçage.

4. Système de sonde interchangeable selon la revendication 1, 2 ou 3, comprenant en outre des moyens de fermeture (27, 227) afin d'empêcher le passage de liquide entre lesdits moyens d'interfaçage et lesdits moyens de raccordement (20, 220).

5. Système de sonde interchangeable selon la revendication 4, dans lequel lesdits moyens de fermeture sont un ensemble formant joint torique (28, 228) comprenant une encoche définie par une partie de surface extérieure desdits moyens d'interfaçage et une seconde gorge définie par une partie de surface intérieure desdits moyens de raccordement (20, 220), ladite encoche et ladite seconde gorge renfermant un joint torique (28, 228), lorsque lesdits moyens de raccordement sont fixés de manière amovible auxdits moyens d'interfaçage, ledit ensemble formant joint torique fournissant une relation fermée et alignée entre lesdits moyens de raccordement et lesdits moyens d'interfaçage.

6. Système de sonde interchangeable selon la revendication 1, comprenant en outre des seconds moyens d'interfaçage disposés à l'extrémité opposée du conduit interne pour permettre la mise en prise desdits moyens de raccordement (20, 220) aux deux extrémités du conduit.

7. Système de sonde interchangeable selon la revendication 1, dans lequel une partie de surface intérieure desdits moyens de réception (25, 225) a un élément fileté femelle.

8. Système de sonde interchangeable selon la revendication 7, dans lequel une partie de surface extérieure de la première extrémité desdits moyens d'adaptateur (24, 224) a un élément fileté mâle (26, 226) pour s'accoupler avec la partie de surface intérieure filetée desdits moyens de réception (25, 225) lorsque lesdits moyens d'adaptateur sont fixés de manière amovible auxdits moyens de réception.
